# EUROPÄISCHE PATENTANMELDUNG

(11) **EP 4 813 323 A1**
(43) Veröffentlichungstag der Anmeldung: **30.09.2026**
(21) Anmeldenummer: 25226889.1
(22) Anmeldetag: 23.12.2025
(51) Int. Cl.: A61B 17/32

(54) **RINGSTANZE ZUR ENTNAHME VON SCHLEIMHAUTTRANSPLANTATEN**

(30) Priorität: 24.03.2025 DE 202025101571 U
(71) Anmelder: Helmut Zepf Medizintechnik GmbH, 78606 Seitingen-Oberflacht (DE)
(72) Erfinder: Zepf, Patrick, 78606 Seitingen-Oberflacht (DE)
(74) Vertreter: Westphal, Mussgnug & Partner Patentanwälte mbB

(57) **Zusammenfassung**

Die Erfindung betrifft eine Ringstanze (10) zur Entnahme von Schleimhauttransplan-taten mit einer äußeren zylindrischen Hülse (20) mit einer Wandung (23), einem ersten Ende (21), einem zweiten Ende (22), einer ersten Längsachse (L1) und einem ersten Innendurchmesser (ID1) sowie mit einer inneren zylindrischen Hülse (30) mit einer Wandung (33), einem ersten Ende (31), einem zweiten Ende, einer zweiten Längsachse (L2) und einem zweiten Innendurchmesser (ID2), wobei die innere zylindrische Hülse (30) konzentrisch in der äußeren zylindrischen Hülse (20) angeordnet ist, und wobei an dem ersten Ende (21) der äußeren zylindrischen Hülse (20) eine erste Schneidkante (25) angeordnet ist und an dem ersten Ende (31) der inneren zylindrischen Hülse (30) eine zweite Schneidkante (35) angeordnet ist, wobei, dass an einer Außenseite der Wandung (23) der äußeren zylindrischen Hülse (20) zumindest abschnittsweise ein radial vorstehender Vorsprung (40) angeordnet ist, welcher zu dem ersten Ende (21) der äußeren zylindrischen Hülse (20) um einen Abstand (A) von mindestens 2 mm und höchstens 4 mm beabstandet ist.

## Beschreibung

Die Erfindung betrifft eine Ringstanze zur Entnahme von Schleimhauttransplantaten gemäß dem Oberbegriff des Patentanspruchs 1.

Als Folge einer Zahnextraktion und Ersetzung des fehlenden Zahns durch ein Zahnimplantat kommt es in der Regel zu einem Remodellierungsprozess der umliegenden Gewebe, und zwar nicht nur auf Knochenebene, sondern auch im Weichgewebe. In vielen Fällen ist dabei auch eine Gewebeaugmentation erforderlich.

Um ein geeignetes Gewebetransplantat, insbesondere Schleimhauttransplantat, an anderer Stelle im Mund entnehmen zu können, sind Ringstanzen zur Entnahme von Schleimhauttransplantaten mit einer äußeren zylindrischen Hülse mit einer Wandung, einem ersten Ende, einem zweiten Ende, einer ersten Längsachse und einem ersten Innendurchmesser sowie mit einer inneren zylindrischen Hülse mit einer Wandung, einem ersten Ende, einem zweiten Ende, einer zweiten Längsachse und einem zweiten Innendurchmesser bekannt, wobei die innere zylindrische Hülse konzentrisch in der äußeren zylindrischen Hülse angeordnet ist, und wobei an dem ersten Ende der äußeren zylindrischen Hülse eine erste Schneidkante angeordnet ist und an dem ersten Ende der inneren zylindrischen Hülse eine zweite Schneidkante angeordnet ist. Mit einer derartigen Ringstanze kann ein ringförmiges Schleimhauttransplantat entnommen und anschließend über den implantierten Zahn gestülpt werden.

Die Aufgabe der Erfindung besteht darin, eine derartige Ringstanze weiterzuentwickeln, so dass sie sicherer einsetzbar ist.

Die Aufgabe wird erfindungsgemäß gelöst durch eine Ringstanze mit den Merkmalen des Patentanspruchs 1.

Vorteilhafte Ausgestaltungen und Weiterbildungen der Erfindung sind in den abhängigen Ansprüchen angegeben.

Bei einer erfindungsgemäßen Ringstanze zur Entnahme von Schleimhauttransplantaten mit einer äußeren zylindrischen Hülse mit einer Wandung, einem ersten Ende, einem zweiten Ende, einer ersten Längsachse und einem ersten Innendurchmesser sowie mit einer inneren zylindrischen Hülse mit einer Wandung, einem ersten Ende, einem zweiten Ende, einer zweiten Längsachse und einem zweiten Innendurchmesser, wobei die innere zylindrische Hülse konzentrisch in der äußeren zylindrischen Hülse angeordnet ist, und wobei an dem ersten Ende der äußeren zylindrischen Hülse eine erste Schneidkante angeordnet ist und an dem ersten Ende der inneren zylindrischen Hülse eine zweite Schneidkante angeordnet ist, ist an einer Außenseite der Wandung der äußeren zylindrischen Hülse zumindest abschnittsweise ein radial vorstehender Vorsprung angeordnet, welcher zu dem ersten Ende der äußeren zylindrischen Hülse um einen Abstand von mindestens 2 mm und höchstens 4 mm beabstandet ist. Ein derartiger radial vorstehender Vorsprung bildet einen Tiefenanschlag, mit welchem eine zu tiefe Gewebeentnahme zuverlässig verhindert werden kann. Dies ermöglicht insbesondere eine sichere Entnahme von Schleimhauttransplantaten auch im Gaumenbereich, ohne dass die Gefahr von Beschädigungen von Blutgefäßen besteht.

Besonders bevorzugt beträgt der Abstand zwischen dem Vorsprung und dem ersten Ende etwa 3 mm, wodurch bei den üblichen anatomischen Ausgestaltungen zuverlässig eine zu tiefe Gewebeentnahme vermieden werden kann.

Gemäß einer besonders bevorzugten Weiterbildung der Erfindung ist der Vorsprung umlaufend ausgebildet, wodurch die Sicherheit weiter erhöht werden kann.

Ist der Vorsprung umlaufend ausgebildet, weist dieser damit eine ringförmige Ausgestaltung auf. Es besteht die Möglichkeit den Vorsprung über die äußere zylindrische Hülse zu stülpen oder an dieser außen anzulegen und den Vorsprung durch Kleben, Pressen oder Lasern zu befestigen.

Vorzugsweise ist jedoch der Vorsprung einstückig an der äußeren zylindrischen Hülse angeordnet. Dadurch ist eine präzisere relative Ausrichtung zwischen Vorsprung und erstem Ende der äußeren zylindrischen Hülse möglich.

Gemäß einer alternativen bevorzugten Ausführungsform ist der Vorsprung verstellbar, insbesondere in Richtung parallel zur ersten Längsachse der äußeren zylindrischen Hülse verschiebbar, an der äußeren zylindrischen Hülse angeordnet. Dadurch kann eine Anpassung an anatomische Gegebenheiten ermöglicht werden. Die Verstellbarkeit kann beispielsweise durch eine Gewindeverbindung, eine Rastverbindung oder eine Klemmverbindung zwischen dem Vorsprung und der äußeren zylindrischen Hülse realisiert werden.

Vorteilhafterweise weist der Vorsprung eine dunkele Färbung auf. Dadurch ist der Vorsprung beim Arbeiten im Mundraum deutlicher erkennbar.

Eine besonders vorteilhafte Ausgestaltung sieht vor, dass in der Wandung der äußeren zylindrischen Hülse in dem Bereich zwischen dem Vorsprung und dem zweiten Ende der äußeren zylindrischen Hülse zumindest eine Öffnung, vorzugsweise zwei diametral gegenüberliegende Öffnungen, angeordnet sind. Die Öffnung mündet somit in den Zwischenraum zwischen der äußeren zylindrischen Hülse und der inneren zylindrischen Hülse, ist aber aufgrund der Anordnung zwischen dem Vorsprung und dem zweiten Ende der äußeren zylindrischen Hülse ausgehend von dem ersten Ende zylindrischen Hülse hinter einem mit der Ringstanze entnommenen Schleimhauttransplantat angeordnet, so dass ein Werkzeug durch die Öffnung in den Zwischenraum eingeführt und durch Druck auf das Schleimhauttransplantat dieses aus der Ringstanze ausgestoßen werden kann.

Da die Ringstanze im Mundraum zur Anwendung kommt, ist die Ringstanze vorteilhafterweise aus Medizinstahl gefertigt, welcher einerseits biokompatibel und andererseits gut reinigbar, insbesondere sterilisierbar ist.

Vorzugsweise ist das zweite Ende der äußeren zylindrischen Hülse und das zweite Ende der inneren zylindrischen Hülse an einem Deckelteil angeordnet, wodurch auf konstruktiv einfache Art und Weise eine relative Fixierung der beiden Hülsen zueinander möglich ist. An der den Hülsen abgewandten Seite des Deckelteils ist ein Antriebsstab angeordnet, welcher vorzugsweise an einem freien Ende einen unrunden Abschnitt aufweist. Der unrunde Abschnitt kann mit einem Handgriff oder einem motorgetrieben Antrieb verbunden werden, um die Ringstanze entweder von Hand oder motorgetrieben um die Längsachse drehen zu können und auf diese Weise das Schleimhauttransplantat einfacher aus dem Gewebe herausschneiden zu können.

Vorzugsweise beträgt die Höhe des radialen Vorsprungs etwa 2 mm beträgt und/oder die axiale Länge des radialen Vorsprungs etwa 1 mm beträgt. Derartige Dimensionen haben sich zur Anwendung der Ringstanze im Mundraum als besonders geeignet erwiesen.

Vorteilhafterweise beträgt der Innendurchmesser der inneren zylindrischen Hülse 3 mm bis 5 mm, vorzugsweise etwa 4 mm. Der Innendurchmesser inneren zylindrischen Hülse bestimmt die Größe des Lochs in dem Schleimhauttransplantat, durch welches das Zahnimplantat, um welchen das Gewebe herum aufgebaut werden soll, ragt. Eine derartige Größe ist für die üblichen Anwendungen gut geeignet.

Vorzugsweise beträgt der Innendurchmesser der äußeren zylindrischen Hülse 6 mm bis 12 mm, beispielsweise etwa 7 mm, etwa 9 mm oder etwa 11 mm, beträgt. Je nachdem, über welchen Bereich um das Zahnimplantat Gewebe herum aufgebaut werden soll, können unterschiedliche Größen zur Anwendung kommen.

Vorzugweise beträgt die Länge der äußeren zylindrischen Hülse etwa 15 mm bis 20 mm, um eine gute Handhabung im Mundraum ermöglichen zu können.

Ein Ausführungsbeispiel der Erfindung wird anhand der nachfolgenden Figuren ausführlich erläutert. Es zeigen
- Fig. 1: eine perspektivische Ansicht eines Ausführungsbeispiels einer erfindungsgemäßen Ringstanze,
- Fig. 2: eine Seitenansicht der Ringstanze gemäß Fig. 1 und
- Fig. 3: eine weitere Seitenansicht der Ringstanze gemäß Fig. 1.

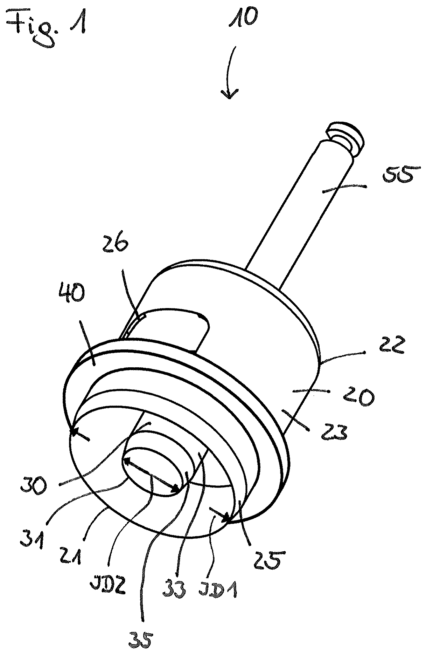

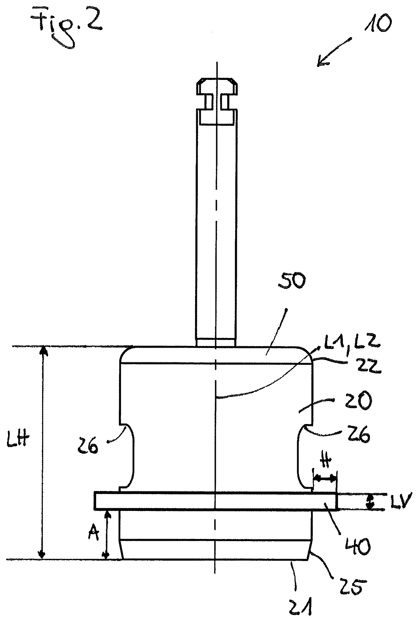

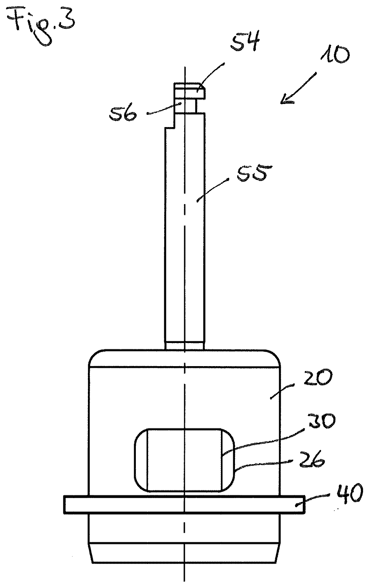

Die Figuren 1 bis 3 zeigen verschiedene Ansichten eines Ausführungsbeispiels einer erfindungsgemäßen Ringstanze 10 zur Entnahme von Schleimhauttransplantaten. Dabei sind zur besseren Übersicht nicht sämtliche Bezugszeichen in sämtlichen Figuren angegeben.

Die Ringstanze 10 weist eine äußere zylindrische Hülse 20 mit einer Wandung 23, einem ersten Ende 21, einem zweiten Ende 22, einer ersten Längsachse L1 und einem ersten Innendurchmesser ID1 sowie eine innere zylindrische Hülse 30 mit einer Wandung 33, einem ersten Ende 31, einem zweiten Ende, einer zweiten Längsachse L2 und einem zweiten Innendurchmesser ID2 auf, wobei die innere zylindrische Hülse 30 konzentrisch in der äußeren zylindrischen Hülse 20 angeordnet. Dadurch fallen insbesondere die erste Längsachse L1 und die zweite Längsachse L2 zusammen.

Der Innendurchmesser ID2 der inneren zylindrischen Hülse 30 kann im Bereich von 3 mm bis 5 mm liegen und vorzugsweise etwa 4 mm betragen. Der Innendurchmesser ID1 der äußeren zylindrischen Hülse 20 kann im Bereich von 6 mm bis 12 mm liegen und beispielsweise etwa 7 mm, etwa 9 mm oder etwa 11 mm betragen. Eine Länge LH der äußeren zylindrischen Hülse kann etwa 15 mm bis 20 mm betragen.

Das zweite Ende 22 der äußeren zylindrischen Hülse 20 und das zweite Ende der inneren zylindrischen Hülse 30 können an einem Deckelteil 50 angeordnet sein. An der den Hülsen 20, 30 abgewandten Seite des Deckelteils 50 kann ein Antriebsstab 55 angeordnet sein, welcher vorzugsweise an einem freien Ende 54 einen unrunden Abschnitt 55 aufweist.

Die beiden Hülsen 20, 30 sind insbesondere derart relativ zueinander angeordnet, dass das erste Ende 21 der äußeren zylindrischen Hülse 20 und das zweite Ende 31 der inneren zylindrischen Hülse 30 in einer Ebene liegen.

An dem ersten Ende 21 der äußeren zylindrischen Hülse 20 ist eine erste Schneidkante 25 angeordnet und an dem ersten Ende 31 der inneren zylindrischen Hülse 30 ist eine zweite Schneidkante 35 angeordnet. Die Schneidkanten 25, 35 können beispielsweise durch eine Fase gebildet sein. Insbesondere ist die Fase jeweils auf einer Außenseite der Wandung 23, 33 der jeweiligen Hülse 20, 30 angeordnet. Die Schneidkante 25, 35 kann sich über eine axiale Länge von etwa 1 bis 2 mm erstrecken.

An einer Außenseite der Wandung 23 der äußeren zylindrischen Hülse 20 ist zumindest abschnittsweise ein radial vorstehender Vorsprung 40 angeordnet, welcher zu dem ersten Ende 21 der äußeren zylindrischen Hülse 20 um einen Abstand A von mindestens 2 mm und höchstens 4 mm, vorzugsweise etwa 3 mm, beabstandet ist. Dieser Vorsprung 40 bildet einen Tiefenanschlag und ermöglicht ein Eindringen der Ringstanze 10 in das Gewebe somit um maximal den Abstand A.

In dem vorliegenden Ausführungsbeispiel ist der Vorsprung 40 umlaufend ausgebildet und weist somit eine ringförmige Ausgestaltung auf. Der Vorsprung 40 kann als separates Teil gefertigt und anschließend auf geeignete Weise an der Außenseite der Wandung 23 befestigt werden. Vorzugsweise ist der Vorsprung 40 jedoch einstückig an der äußeren zylindrischen Hülse 20 angeordnet. Der Vorsprung 40 weist eine Höhe H auf, welche etwa 2 mm betragen kann. Der Vorsprung kann sich über eine axiale Länge LV von etwa 1 mm erstrecken. Weiterhin kann der Vorsprung 40 eine dunkele Färbung aufweisen, um ihn im Mundraum besser erkennen zu können.

In der Wandung 23 der äußeren zylindrischen Hülse 20 können in dem Bereich zwischen dem Vorsprung 40 und dem zweiten Ende 22 zumindest eine Öffnung 26, vorliegen zwei diametral gegenüberliegende Öffnungen 26, angeordnet sein. Dabei ist es vorteilhaft, wenn die Öffnung 26, insbesondere beide Öffnungen 26, möglichst nahe an dem Vorsprung 40 angeordnet sind. Durch diese Öffnungen 26 kann ein Werkzeug in den Zwischenraum zwischen der äußeren zylindrischen Hülse 20 und der inneren zylindrischen Hülse 30 eingeführt und dadurch ein sich in diesem Zwischenraum befindliches Schleimhauttransplantat zum ersten Ende 21 der äußeren zylindrischen Hülse 20 hin aus der Ringstanze 10 herausgeschoben werden.

Die Ringstanze 10 ist aus Medizinstahl gefertigt, zum Beispiel aus einem martensitischen Edelstahl.

### Bezugszeichenliste

- 10: Ringstanze
- 20: äußere zylindrische Hülse
- 21: erstes Ende
- 22: zweites Ende
- 23: Wandung
- 25: erste Schneidkante
- 26: Öffnung
- 30: innere zylindrische Hülse
- 31: erstes Ende
- 33: Wandung
- 35: zweite Schneidkante
- 40: Vorsprung
- 40: Deckelteil
- 54: freies Ende
- 55: Antriebsstab
- 56: unrunder Abschnitt
- ID1: erster Innendurchmesser
- ID2: zweiter Innendurchmesser
- L1: erste Längsachse
- L2: zweite Längsachse
- A: Abstand
- H: Höhe
- LV: Länge
- LH: Länge

## Patentansprüche

1. Ringstanze (10) zur Entnahme von Schleimhauttransplantaten mit einer äußeren zylindrischen Hülse (20) mit einer Wandung (23), einem ersten Ende (21), einem zweiten Ende (22), einer ersten Längsachse (L1) und einem ersten Innendurchmesser (ID1) sowie mit einer inneren zylindrischen Hülse (30) mit einer Wandung (33), einem ersten Ende (31), einem zweiten Ende, einer zweiten Längsachse (L2) und einem zweiten Innendurchmesser (ID2), wobei die innere zylindrische Hülse (30) konzentrisch in der äußeren zylindrischen Hülse (20) angeordnet ist, und wobei an dem ersten Ende (21) der äußeren zylindrischen Hülse (20) eine erste Schneidkante (25) angeordnet ist und an dem ersten Ende (31) der inneren zylindrischen Hülse (30) eine zweite Schneidkante (35) angeordnet ist,
**dadurch gekennzeichnet, dass** an einer Außenseite der Wandung (23) der äußeren zylindrischen Hülse (20) zumindest abschnittsweise ein radial vorstehender Vorsprung (40) angeordnet ist, welcher zu dem ersten Ende (21) der äußeren zylindrischen Hülse (20) um einen Abstand (A) von mindestens 2 mm und höchstens 4 mm beabstandet ist.

2. Ringstanze nach Anspruch 1,
**dadurch gekennzeichnet, dass** der Abstand (A) zwischen dem Vorsprung (40) und dem ersten Ende (21) etwa 3 mm beträgt.

3. Ringstanze nach einem der vorherstehenden Ansprüche,
**dadurch gekennzeichnet, dass** der Vorsprung (40) umlaufend ausgebildet ist.

4. Ringstanze nach einem der vorherstehenden Ansprüche, **dadurch gekennzeichnet, dass** der Vorsprung (40) einstückig an der äußeren zylindrischen Hülse (20) angeordnet ist.

5. Ringstanze nach einem der Ansprüche 1 bis 3,
**dadurch gekennzeichnet, dass** der Vorsprung (40) verstellbar an der äußeren zylindrischen Hülse (20) angeordnet ist.

6. Ringstanze nach einem der vorherstehenden Ansprüche, **dadurch gekennzeichnet, dass** der Vorsprung (40) eine dunkele Färbung aufweist.

7. Ringstanze nach einem der vorherstehenden Ansprüche, **dadurch gekennzeichnet, dass** in der Wandung (23) der äußeren zylindrischen Hülse (20) in dem Bereich zwischen dem Vorsprung (40) und dem zweiten Ende (22) zumindest eine Öffnung (26), vorzugsweise zwei diametral gegenüberliegende Öffnungen (26), angeordnet sind.

8. Ringstanze nach einem der vorherstehenden Ansprüche, **dadurch gekennzeichnet, dass** die Ringstanze (10) aus Medizinstahl gefertigt ist.

9. Ringstanze nach einem der vorherstehenden Ansprüche, **dadurch gekennzeichnet, dass** das zweite Ende (22) der äußeren zylindrischen Hülse (20) und das zweite Ende der inneren zylindrischen Hülse (30) an einem Deckelteil (50) angeordnet ist, wobei an der den Hülsen (20, 30) abgewandten Seite des Deckelteils (50) ein Antriebsstab (55) angeordnet ist, welcher vorzugsweise an einem freien Ende (54) einen unrunden Abschnitt (56) aufweist.

10. Ringstanze nach einem der vorherstehenden Ansprüche, **dadurch gekennzeichnet, dass** die Höhe (H) des radialen Vorsprungs (40) etwa 2 mm beträgt und/oder die axiale Länge (LV) des radialen Vorsprungs (40) etwa 1 mm beträgt.

11. Ringstanze nach einem der vorherstehenden Ansprüche, **dadurch gekennzeichnet, dass** der Innendurchmesser (ID2) der inneren zylindrischen Hülse (30) 3 mm bis 5 mm, vorzugsweise etwa 4 mm beträgt.

12. Ringstanze nach einem der vorherstehenden Ansprüche, **dadurch gekennzeichnet, dass** der Innendurchmesser (ID1) der äußeren zylindrischen Hülse (20) 6 mm bis 12 mm, beispielsweise etwa 7 mm, etwa 9 mm oder etwa 11 mm, beträgt.

13. Ringstanze nach einem der vorherstehenden Ansprüche, **dadurch gekennzeichnet, dass** die Länge (LV) der äußeren zylindrischen Hülse (20) etwa 15 mm bis 20 mm beträgt.
